# EUROPÄISCHE PATENTSCHRIFT

(11) **EP 3 269 340 B1**
(45) Veröffentlichungstag und Bekanntmachung des Hinweises auf die Patenterteilung: **10.07.2024**
(21) Anmeldenummer: 16179093.6
(22) Anmeldetag: 12.07.2016
(51) Int. Cl.: A61F 7/02

(54) **VORRICHTUNG ZUR HYPERTHERMISCHEN BEHANDLUNG VON PRURITUS**
DEVICE FOR HYPERTHERMY TREATMENT OF PRURITUS
DISPOSITIF DE TRAITEMENT HYPER-THERMIQUE DE PRURIT

(43) Veröffentlichungstag der Anmeldung: 17.01.2018
(73) Patentinhaber: Dermapharm AG, 82031 Grünwald (DE)
(72) Erfinder: ZIPPENPFENNIG, Jörg, 18519 Sundhagen (DE)
(74) Vertreter: Hertin und Partner Rechts- und Patentanwälte PartG mbB

(56) Entgegenhaltungen:
- EP-A1- 2 206 483
- WO-A1-01/34074
- WO-A1-2007/082648
- WO-A2-2006/063202
- US-B1- 6 245 093

## Beschreibung

Die Erfindung betrifft eine Vorrichtung nach Anspruch 1. Die erfindungsgemäße Vorrichtung erlaubt ein starkes Lindern des Juckreizes über großflächige Hautpartien und eine effektive Behandlung von Pruritus, chronischem Pruritus, Dermatitis, Allergien oder Vergiftungen durch Nesseltiere.

### Hintergrund und Stand der Technik

Juckreiz (Pruritus) ist eine subjektiv unangenehme, auf die Haut oder Schleimhaut bezogene Sinneswahrnehmung. Sie kann lokal begrenzt sein oder aber den ganzen Körper betreffen.

Häufig geht Juckreiz mit einem brennenden, stechenden oder kribbelnden Gefühl einher, welches die betroffene Person oftmals durch Kratzen, Scheuern, Rubbeln, Drücken, Kneten oder Reiben versucht zu lindern. Daher kommt es bei Juckreiz gehäuft zu weiteren pathologischen Erscheinung der Haut wie Kratzspuren, offene Wunden, Krustenbildung und Hautinfektionen. Die Fachwelt geht davon aus, dass Juckreiz über Schmerzrezeptoren der Haut vermittelt und über das vegetative Nervensystem zum Gehirn geleitet wird. Die Ursachen von Juckreiz können sehr vielfältig sein. Neben trockener Haut, fehlender Feuchtigkeitszufuhr oder Allergien kann Juckreiz auch durch äußere Einwirkungen und Hautreizungen, wie z.B. durch Stiche von Mücken oder nach Kontakt mit Nesseltieren entstehen. Juckreiz kann eine Reaktion auf chemische, mechanische oder thermische Reize sein. Es können als Folge der äußerlichen Reizung, wie z.B. durch Einwirkungen chemischer Substanzen, z.B. Histamin (Mückenstich), Apamin (Bienenstich), durch allergische Immunreaktion, durch Druck oder Reibung oder auch durch Wärme oder Sonnenbestrahlung, Quaddeln, Urtikaria und andere mit Juckreiz verbundene Hautreaktionen hervorgerufen werden. Aus medizinischer Sicht spannen die Ursachen oder Grunderkrankungen, welche zu Juckreiz führen ein breites Spektrum dermatologischer und internistischer Erkrankungen auf.

Hierzu gehören Hauterkrankungen wie Ekzeme, Neurodermitis, Urtikaria, Hautinfektionen (z.B. Candidose), Xerodermie, Parasiten (z.B. Skabies), Insektenstiche oder Allergien, Stoffwechselerkrankungen wie u.a. Eisenmangelanämie oder Diabetes mellitus, Infektionskrankheiten insbesondere AIDS, Varizellen, Masern oder Herpes Zoster, proktologische Erkrankungen wie u.a. Hämorrhoiden oder Analekzem, Nierenerkrankungen wie Urämie, Lebererkrankungen und Leberinsuffizienz oder Neoplasie insbesondere Mycosis fungoides oder Morbus Hodgkin. Auch hormonelle Ursachen wie eine Menopause oder Nebenwirkungen durch Medikamenteneinnahme insbesondere von Carbamazepin, Antibiotika, Opiate (Morphium), Hydroxyethylstärke, Captopril und Miconazol können Pruritus auslösen und fördern. Weiterhin sei der senile Pruritus genannt, welcher im Alter zu unangenehmen Hautreizungen führt.

Chronischer Pruritus (ständiger Juckreiz) stellt einen besonders schwerwiegenden Juckreiz dar, welcher als äußerst schwer behandelbares Symptom insbesondere bei o.g. Grunderkrankungen auftritt (Rothmann et al. 1941.). Pruritus wird als chronisch bezeichnet, wenn er anhaltend über sechs Wochen besteht. Die Punktprävalenz von chronischem Pruritus beträgt bis zu 13,5 % in der Allgemeinbevölkerung (Ständer et al. 2007) und bis zu 16,8 % in der arbeitenden Bevölkerung (Ständer et al. 2010). Auch ein gehäuftes Auftreten von Neuerkrankungen ist zu verzeichnen. Die Inzidenz von Pruritus beträgt 7 % in einem Jahr (Vogelgesang et al. 2012).

Neben der physischen und psychologischen Belastung der Betroffenen, stellen die medizinischen Kosten und Arbeitsausfälle durch insbesondere chronischen Pruritus eine wesentliche sozio-ökonomische Belastung für die Gesellschaft dar. Die Weiterentwicklung von Therapien ist daher von hoher Relevanz.

Grundsätzlich ist eine Behandlung der Grunderkrankung die wesentliche Säule für eine nachhaltige Therapie von Pruritus. Falls die Ursache bekannt ist, kann bspw. eine spezifische Behandlung der Dermatose, eine Meidung des identifizierten Kontaktallergens, das Absetzen eines Medikamentes oder eine spezifische internistische, neurologische, psychiatrische oder auch operative (z.B. im Falle eines Tumors) Behandlung erfolgen. Die Besserungschancen des Pruritus sind bei einer effektiven Behandlung der Grunderkrankung als positiv zu bewerten. Häufig ist die Ursache des Pruritus jedoch unbekannt oder kaum behandelbar. Die Diversität und Komplexität der möglichen Ursachen erschweren weiterhin eine ursächlich-angepasste Therapie.

Zur medikamentösen Behandlung von den Symptomen des Juckreizes sind eine Reihe von Medikamenten oder kosmetischen Produkten bekannt. So werden ätherische Öle insbesondere umfassend Menthol, Thymol oder Campher verwandt, um kurzfristig eine Kühlung zu erzeugen. Auch Hautpflegemittel wie beispielsweise Cremen oder Lotion können durch eine Erhöhung des Feuchtigkeitsgehaltes der Haut eine schmerzlindernde Wirkung entfalten. Darüber hinaus stellen Antihistaminika hilfreiche Therapiemöglichkeiten dar, welche beispielsweise die Gabe von Dimetindenmaleat oder Mepyramin umfassen. Weitere Medikamente betreffen topische Glucocorticoide, Anästhetika, Zinksalben, Calcineurinhemmer oder Capsaicin.

Bei der medikamentösen Behandlung von Pruritus ist jedoch zu beachten, dass diese zu Nebenwirkungen insbesondere in Kombination mit Medikamenten zur Behandlung der Grunderkrankung führen können. Weiterhin stellt bei Pruritus das breite Patientenkollektiv, welches von Kindern über Schwangeren bis hin zu multimorbiden Patienten reicht, eine therapeutische Herausforderung dar. Eine medikamentöse Behandlung von Pruritus führt oftmals nicht zum gewünschten Erfolg oder geht mit Nebenwirkungen einher. Aus diesem Grunde ist die Entwicklung alternativer Therapien zur Behandlung von Pruritus erforderlich, welche nicht auf der Gabe von Medikamenten beruhen.

Aus dem Stand der Technik ist bekannt durch Einbringung einer Wärmemenge in den Einstich von Insekten das Auslösen eines Juckreizes zu mindern. Eine Vorrichtung für eine lokale, thermische Behandlung insbesondere von Mückenstichen wird in der EP 1231875 B1 beschrieben. Die Vorrichtung weist eine Heizplatte mit einer Größe von ca. 0,2 cm² auf, welche auf eine Temperatur zwischen 50°C und 65°C gebracht wird, während die Heizplatte den Insektenstich kontaktiert. Während sich das Gerät gut für die Linderung von Juckreiz bei kleineren Insektenstichen geeignet, kann es nicht bei großflächigen Hautreizungen im Falle von chronischem Pruritus oder bei Kontakt mit Nesseltieren verwandt werden. Für diese Erkrankungen wird im Stand der Technik auf konventionelle medikamentöse Behandlungen mit den o.g. Nachteilen zurückgegriffen.

Weitere relevante Vorrichtungen und Verfahren sind in dem Stand der Technik in US 6 245 093 B1, EP 2 206 483 A1, WO 2006/063202 A2, WO 2007/082648 A1 und WO 01/34074 A1 offenbart.

### Aufgabe der Erfindung

Aufgabe der Erfindung war es eine Vorrichtung bereitzustellen, welche die Nachteile des Standes der Technik beseitigt. Insbesondere sollte eine Vorrichtung bereitgestellt werden, welche sich zur Behandlung von großflächigem, auch chronischem Pruritus eignet und eine höhere Effektivität und Sicherheit gegenüber bekannten Vorrichtungen und Verfahren aufweist.

### Zusammenfassung der Erfindung

Die Aufgabe der Erfindung wird durch eine Vorrichtung gemäß des unabhängigen Anspruches 1 gelöst. Die abhängigen Ansprüche betreffend bevorzugte Ausführungsformen der Erfindung.

Die Erfindung betrifft eine Vorrichtung zur hyperthermischen Behandlung von Juckreiz umfassend
a) eine Behandlungsfläche
b) mindestens eine Heizplatte
   und
b) eine Steuervorrichtung zur Regulation der Temperatur der Behandlungsfläche wobei
die Größe der Behandlungsfläche zwischen 6 cm² und 18 cm² beträgt und die Steuervorrichtung konfiguriert ist die Temperatur der mindestens einen Heizplatte (5) zu regulieren, sodass die die Behandlungsfläche in einer Aufheizphase auf eine Behandlungstemperatur zwischen 42°C und 53°C reguliert wird und die Behandlungstemperatur in einer Behandlungsphase für einen zusammenhängenden Zeitraum von 4 s bis 6 s gehalten wird.

Zur Linderung des Juckreizes wird die erfindungsgemäße Vorrichtung bevorzugt auf die zu behandelnden Hautpartien des Patienten aufgelegt. Sobald die Vorrichtung mit der Behandlungsfläche die betreffenden Hautstellen kontaktiert, kann mit Hilfe der Steuervorrichtung eine erfindungsgemäße Regulation der Temperatur der Behandlungsfläche vorgenommen werden. Hierzu wird zunächst in einer Aufheizphase die Behandlungsfläche auf eine Behandlungstemperatur zwischen 42°C und 53°C geführt. Es ist bevorzugt, dass die Aufheizphase kurz gehalten wird. Bevorzugt sollte die Aufheizphase nicht mehr als 10 s, besonders bevorzugt nicht mehr als 2 s betragen. Nach vollendeter Aufheizphase wird die Temperatur der Behandlungsfläche bevorzugt bei der vorbestimmten Behandlungstemperatur gehalten. Die Behandlungstemperatur entspricht bevorzugt einer konstanten Temperatur, welche im genannten Bereich zwischen 42°C und 53°C liegt. Diese Behandlungstemperatur wird während der Behandlungsphase bevorzugt konstant gehalten. Die Behandlungsphase bezeichnet den Zeitraum, bei welchem die Temperatur im Bereich der Behandlungstemperatur von 42°C bis 53°C ist. Erfindungsgemäß dauert die Behandlungsphase zwischen 4 s und 6 s. Die Behandlungsphase bezeichnet einen zusammenhängenden Zeitraum bezeichnet.

Durch die Regulation der Behandlungsfläche auf einer Temperatur zwischen 42°C und 53°C für eine Behandlungsphase zwischen 4s und 6 s, wird ein Hitzeimpuls generiert, welcher es erlaubt eine wohldefinierte Wärmemenge auf die Hautstelle auf kontrollierte Weise zu bringen. Die Wärmeübertragung führt überraschend zu einer Überlagerung des Juckreizes durch andere temperaturabhängige Hautempfindungen. Entgegen konventionellen Methoden zur Behandlung von Pruritus, welche das Juckempfinden über eine Regulation der Schmerzrezeptoren anvisieren, wird durch die erfindungsgemäße Wärmebehandlung die freien Nervenenden der C-Fasern aktiviert. Die C-Fasern bezeichnen insbesondere die langsam leitenden Nervenfasern des somatosensiblen Systems und sind für die Schmerzwahrnehmung verantwortlich. Hierbei kommt insbesondere den freien Enden der C-Fasern, welche auch als Nozizrezeptoren bezeichnet werden, eine wichtige Rolle zu. Die Nervenenden der Fasern werden durch Gewebshormone (z. B. Histamin, Serotonin, Substanz P) aktiviert. Auch könnten Mastzellen in der Nähe der Nervenenden durch Ausschüttung des Mediators Tryptase an dem Prozess beteiligt sein.

Erfindungsgemäß wird die Erkenntnis über den Wirkmechanismus bei Pruritus ausgenutzt, um durch eine Wärmebehandlung in überraschender Weise die Sinneswahrnehmung, welche durch die Fasern ausgelöst werden, zu regulieren. Im Stand der Technik sind zwar kleinere Heizplatten zur Behandlung von Insektenstichen bekannt. Ein Fachmann konnte jedoch nicht davon ausgehen, dass eine Wärmebehandlung auch bei großflächig betroffen Hautpartien wie im Falle von chronischem Pruritus, Allergien oder Quallenstichen vorteilhaft ist.

So geht die Fachwelt davon aus, dass die hyperthermische Behandlung von Mückenstichen auf einer Inhibierung des Insektengiftes beruht, wodurch der Juckreiz gelindert wird. Die erfindungsgemäße Erkenntnis des obig ausgeführten Wirkmechanismus, lässt jedoch eine weiterführende Anwendung auch auf großflächige Hautirritation zu. Hierbei kann Pruritus ganz unterschiedlicher Ursachen effektiv gelindert werden. Weiterhin stellt die Konzeption einer größeren Behandlungsfläche eine drastische Abkehr vom Stand der Technik für die hyperthermische Behandlung von Juckreiz dar. So ging die Fachwelt davon aus, dass bei einer großflächigen Behandlung betroffener Hautpartien, eine positive Linderung des Juckreizes durch starke negative Nebenwirkungen wie Hautverbrennung oder hyperthermisches Schmerzempfinden überlagert werden. Erfindungsgemäß wurde erkannt, dass mit einer grö-ßeren Behandlungsfläche von 6 cm² bis 18 cm² auf überraschender Weise auch großflächige Hautpartien, welche von Pruritus betroffen sind, behandelt werden können. So ist es beispielsweise möglich im Falle von Hautauschlägen durch bequemes und einfaches Auflegen der Behandlungsfläche auf die entsprechenden Hautpartien, das Juckempfinden durch die Hitzeübertragung in eine erträgliche Schmerzempfindung zu überführen. Sekundäre Schädigungen der Haut, beispielsweise Wundbildungen durch starkes Kratzen, können so wirksam vermieden werden. Mit Vorrichtungen des bekannten Standes der Technik wäre für eine Behandlung von großflächigen Hautpartien ein mehrmaliges Auflegen an unterschiedlichen Positionen notwendig. Aufgrund des Zeitversatzes kann hierdurch jedoch nicht derselbe Effekt erzielt werden kann.

Im Sinne der Erfindung bezeichnet die "Behandlungsfläche" diejenige Fläche der Vorrichtung, welche während der Behandlung auf die Behandlungstemperatur aufgeheizt wird und in direktem thermischen Kontakt mit der Hautpartie steht. Die Behandlungsfläche kann eine zusammenhängende Fläche darstellen. Es kann auch bevorzugt sein, dass die Behandlungsfläche aus mehreren nicht-zusammenhängenden Teilflächen besteht. Die Größe der Behandlungsfläche bezieht sich jeweils auf die gesamte Kontaktfläche. Im Falle einer Behandlungsfläche, welche aus mehreren Teilflächen besteht, entspricht die Größe der Behandlungsfläche nicht etwa der Umrissfläche, welche die Teilflächen einschließt, sondern vielmehr der Summe der einzelnen Teilflächen. Die Umrissfläche entspricht bevorzugt der konvexen Hülle der Teilflächen gemäß der mathematischen Definition. Im Falle einer zusammenhängenden Behandlungsfläche ist die Größe der Behandlungsfläche gleich der Umrissfläche.

Bevorzugt wird die Behandlungsfläche mit Hilfe von mindestens einer Heizplatte auf die Behandlungstemperatur gebracht. Die Behandlungsfläche liegt hierzu bevorzugt, in Kontakt mit der mindestens einen Heizplatte, deren Temperatur durch die Steuervorrichtung eingestellt werden kann, wobei durch ein Erhitzen der Heizplatte eine Einstellung der Behandlungstemperatur an der Behandlungsfläche erfolgt. Im Sinne der Erfindung ist die Steuervorrichtung bevorzugt ein Prozessor oder ein Prozessorchip, welcher konfiguriert ist, um die Temperatur der Heizplatten gemäß vorgegebener Werte zu regulieren. Die mindestens eine Heizplatte ist bevorzugt ein Bauelement, wie es aus dem Stand der Technik hinreichend bekannt ist. Es ist bevorzugt, dass die Heizplatte durch Anlegung einer Spannung auf eine gewünschte Temperatur geführt werden kann, aber auch andere Mittel zur Wärmebereitstellung können vorgesehen sein. Bevorzugt wird unter der Heizplatten jenes Bauelement bezeichnet, welches durch die Steuervorrichtung u.a. durch Anlegen eines elektrischen Stromes erhitzt werden kann. Es ist jedoch bevorzugt, dass die Heizplatte nicht direkt mit der zu behandelnden Haut in Berührung kommt. Zu diesem Zweck befindet sich die Heizplatte bevorzugt oberhalb der Behandlungsfläche und steht in direktem oder indirektem thermischen Kontakt mit der Behandlungsfläche. Oberhalb bezeichnet bevorzugt die nach innen gerichtete Positionierung der Heizplatte, welche außen von einer sich unterhalb der Heizplatte befindenden Behandlungsfläche ummantelt ist. Der direkte thermische Kontakt bezeichnet bevorzugt einen unmittelbaren Kontakt und der indirekte thermische Kontakt einen Kontakt, welcher über eine wärmeleitende Schicht realisiert wird. Es kann sowohl bevorzugt sein, dass die Vorrichtung eine einzige Heizplatte aufweist. Es kann aber auch bevorzugt sein, dass die Vorrichtung mehrere Heizplatten umfasst.

Erfindungsgemäß beträgt die Größe der Behandlungsfläche mindestens 6 cm² und die Behandlungstemperatur liegt zwischen 42°C und 53°C, besonders bevorzugt zwischen 50°C und 53°C.

Es hat sich völlig überraschend gezeigt, dass mit den vorgenannten Parametern ein Juckreiz insbesondere auf großflächigen Hautpartien besonders stark reduziert werden kann. Eine Kombination einer Behandlungsfläche von mindestens 6 cm² mit einer Behandlungstemperatur von 42°C und 53°C und insbesondere mit der bevorzugten Behandlungstemperatur zwischen 50°C und 53°C erlaubt eine Wirkung auf die Hautpartien, welche den Juckreiz schnell und effektiv lindern. Erfindungsgemäß erkannt, dass eine besonders starke Überlagerung des Juckempfindens erreicht werden kann, wenn in den betreffenden Hautpartien lokal die Thermo- und Capsaicinrezeptoren TRPV1 und TRPV2 zeitgleich aktiviert werden.

Der TRPV1 ist bei akutem Hitze-induzierten Schmerz in gesunder Haut beteiligt und reguliert beispielsweise das Heißempfinden bei Temperaturen um 45° bis 50 °C. Bei besonders starken schmerzhaften Hitzereizen, welche ab Temperaturen von über 52 °C eintreten wird darüber hinaus der TRPV2 aktiviert. Die Aktivierungsschwelle des TRPV1 liegt zwischen 40°C und 45°C, wohingegen die des TRPV2 zwischen 50°C und 53° beträgt (Yao et al 2011, Somogyi et al. 2015, Cohen et al. 2014, Mergler et al. 2014).

Während durch aktuelle Forschungsergebnisse in der Literatur ein erstes Verständnis über die Wirkungsweise der TRPV1 und TRPV2 Rezeptoren als Temperatursensoren aufkommt, ist deren Rolle in der Empfindung von Juckreizen unbekannt. Ein Fachmann würde daher auch unter Kenntnis der Literatur nicht davon ausgehen, dass gerade die Aktivierung dieser Rezeptoren eine besonders effektive Überlagerung des Juckempfindens ermöglicht. Dies ist eine überraschende Erkenntnis, welche erfindungsgemäß gewonnen wurde. Die bevorzugt genannte Ausführungsform, welche eine Temperaturregelung der Behandlungsfläche in einem engen Bereich zwischen 50°C und 53°C vorsieht, erlaubt auf überraschend effektive Weise eine zeitgleiche Aktivierung der Rezeptoren, ohne unangenehm starke Schmerzempfindungen bei den behandelten Personen auszulösen. Durch experimentelle Versuche konnte der Bereich der Aktivierungsschwelle des TRPV2 als besonders optimierter Wirkbereich ermittelt werden. Hierbei kommt es voraussichtlich zu einem Feedbackmechanismus zwischen den Rezeptoren, welcher den Juckreiz besonders effektiv überlagert, ohne Nebenwirkungen zu verursachen. Für eine Behandlungsfläche, welche größer als 6 cm² ist, konnte ein Fachmann dies nicht erwarten. Vielmehr wäre ein Fachmann davon ausgegangen, dass bei einer Behandlungstemperatur 50°C - 53°C über einen Zeitraum von 2 s bis 12 s und erst recht erfindungsgemäßen Zeitraum von 4s bis 6 s zu starken Hautirritationen oder Schmerzen bis hin zu leichten Verbrennungen kommt. Stattdessen führt die bevorzugte Behandlung der Hautpartien zu einem Abmildern des Juckempfindens, welches überraschenderweise noch Stunden nach der Behandlung anhält. Die langanhaltende Wirkungsweise der bevorzugten Ausführungsform ist zu mindestens teilweise auf eine Immunregulation durch die Wärmübertragung zurückzuführen. So wird nicht nur das Schmerzempfinden überlagert, sondern durch eine Regulation des Immunsystems wird die lokale Reizung der Haut aktiv unterdrückt. Vorteilhafterweise kann daher eine einmalige Behandlung bereits zu einem nachhaltigen Nachlassen des Juckempfindens führen. Es kann aber auch bevorzugt sein, mehrmals in zeitlicher Abfolge eine Behandlung durchzuführen. Die intervallartige Übertragung der Wärme mit einer Behandlungsphase von 4 s bis 6 s erreicht eine optimale Wirkung auf die Signalwege des Pruritus ohne unerwünschte Nebenwirkungen auszulösen.

In einer bevorzugten Ausführungsform der Erfindung ist die Vorrichtung dadurch gekennzeichnet, dass die Vorrichtung mindestens zwei Heizplatten, bevorzugt mindestens vier Heizplatten und ganz besonders bevorzugt mindestens sechs Heizplatten umfasst, welche in Kontakt mit der Behandlungsfläche vorliegen und deren Temperatur durch die Steuervorrichtung eingestellt werden kann, wobei durch ein Erhitzen der Heizplatten eine Einstellung der Behandlungstemperatur an der Behandlungsfläche erfolgt. Durch das Vorhandensein von zwei Heizplatten, deren Positionierung frei wählbar ist, kann eine besonders vorteilhafte Temperaturverteilung auf der Haut des Patienten erreicht werden. So kann es bevorzugt sein, dass die Behandlungsfläche eine zusammenhängende Fläche ist, über welcher die mindestens zwei, bevorzugt mindestens vier, besonders bevorzugt mindestens sechs Heizplatten angebracht vorliegen. Hierdurch ist es möglich eine optimierte Temperaturverteilung zu erreichen.

Zu diesem Zweck können die Abstände der Heizplatten angepasst werden. Beispielsweise kann es bevorzugt sein, dass die Heizplatten gitterförmig angeordnet werden, wobei zwischen den Heizplatten jeweils ein Abstand besteht, welcher zwischen 0,2 mm und 10 mm, bevorzugt zwischen 0,5 mm und 3 mm, beträgt. Hierfür kann eine besonders wirkungsvolle Temperaturverteilung generiert werden. So ist die Temperaturverteilung nicht exakt homogen, sondern an der Positionierung der Heizplatten leicht, d.h. bevorzugt um weniger als 2°C, erhöht. Die Temperaturdifferenz wird bevorzugt im Vergleich zu einer niedrigen Temperatur der Behandlungsfläche zwischen den Heizplatten angegeben. Überraschenderweise führt eine derart lokal geringfügige Heterogenität mit einer Temperaturdifferenz zwischen 0,1°C bis 2°C bezogen auf die durchschnittliche Behandlungstemperatur zu einem besonders ausgeprägten Lindern des Juckreizes. Die Ausdehnung bezeichnet bevorzugt eine charakteristische Länge des Querschnittes der Heizplatten. Im Falle von kreisförmigen Heizplatten entspricht die Ausdehnung bevorzugt dem Durchmesser. Im Fall von quadratförmigen Heizplatten ist unter der Ausdehnung bevorzugt die Seitenlänge des Quadrates zu verstehen. Es kann bevorzugt sein, dass zur Reduzierung des Ansteuerungsaufwandes die Vorrichtung nicht mehr als 15 bevorzugt nicht mehr als 12 Heizplatten aufweist.

Es kann aber auch bevorzugt sein, dass die Behandlungsfläche aus mehreren Teilflächen besteht, welche bevorzugt kongruent zu den Heizplatten sind. Kongruent meint bevorzugt, eine Deckungsgleichheit der Teilflächen der Behandlungsfläche und der sich darüber befindenden Heizplatten, sodass die Form und Größe der Teilflächen der Form und Größe der Heizplatten entspricht. Hierzu können beispielsweise die Heizplatten mit dem für die Behandlungsfläche bevorzugt verwandten Materialien beschichtet oder überzogen werden. Es kann aber auch bevorzugt sein, dass die Teilflächen die Heizplatten überragen, wobei es bevorzugt ist, dass die Teilflächen der Behandlungsfläche einander in der Kontaktebene nicht berühren.

Es ist bevorzugt, dass die Behandlungsfläche aus mehreren Teilflächen besteht, wenn beispielsweise besonders großflächige Hautstellen behandelt werden sollen. Zu diesem Zweck können die Teilflächen derart angeordnet werden, dass sie weit auseinanderliegen, um eine große Umrissfläche abzudecken. Beispielsweise kann es bevorzugt sein, dass die Teilflächen gitterförmig verteilt werden, wobei zwischen den Teilflächen jeweils ein Abstand besteht, welcher zwischen 0,2 mm und 10 mm, bevorzugt zwischen 0,5 mm und 3 mm, beträgt. Beim Auflegen der Behandlungsfläche auf die Hautpartien wird im Vergleich zu einer zusammenhängenden Behandlungsfläche eine stärker heterogene Temperaturverteilung erreicht, welches sich bei bestimmten Pruritus-Erkrankungen vorteilhaft auswirken kann. Weiterhin wird die insgesamt übertragene Wärmemenge durch eine punktuelle Hitzeabgabge innerhalb der Umrissfläche vorteilhafterweise reduziert.

Es ist besonders bevorzugt dass die Heizplatten durch die Steuervorrichtung auf eine Temperatur eingestellt werden, welche an der Behandlungsfläche zu der gewünschten Behandlungstemperatur führt. So kann es bevorzugt sein, dass die Heizplatten im Vergleich zur anvisierten Behandlungstemperatur auf eine leicht erhöhte Temperatur gesteuert werden. Die Differenz der Temperaturen reflektiert den erwartbaren Temperaturgradienten zwischen der Heizplatte und der Außenseite der Behandlungsfläche.

In einer bevorzugten Ausführungsform der Erfindung ist die Vorrichtung dadurch gekennzeichnet, dass die Steuervorrichtung die Heizplatten in der Aufheizphase auf eine Heiztemperatur zwischen 43°C und 54°C regulieren kann und die Heiztemperatur in der Behandlungsphase für einen Zeitraum von 4 sec und 6 s gehalten werden kann. Die Heiztemperatur entspricht somit bevorzugt jener Temperatur auf welcher die Heizplatten bevorzugt während der Behandlungsphase konstant gehalten werden. Durch eine derartige Regulation der Heizplatten kann die Temperatur der Behandlungsfläche besonders präzise in einem Bereich von 42°C - 53 °C geführt werden, mit einem entsprechenden Zeitraum der Behandlungsphase 4 s und 6 s. Es kann dabei bevorzugt sein, dass die Heizplatten auf eine leicht erhöhte Temperatur eingestellt werden, als die gewünschte Behandlungstemperatur, um eventuelle Wärmeverluste und einen Temperaturgradienten auszugleichen. Weiterhin kann es bevorzugt sein, dass die Heizplatten konstant auf die gleiche Heiztemperatur eingestellt werden. Es kann aber auch bevorzugt sein, dass die Heizplatten auf unterschiedliche Temperaturen eingestellt werden. Es ist daher bevorzugt, dass die Heizplatten über die Steuereinrichtung separat angesteuert und erhitzt werden können. Insbesondere für Ausführungsformen mit mindestens 6 oder bevorzugt mindestens 8 Heizplatten hat sich gezeigt, dass eine überraschend homogene Temperaturverteilung auf der Behandlungsfläche erzielt werden kann, wenn die sich am Rand befindenden Heizplatten auf eine leicht erhöhte Temperatur erhitzt werden, als mittlere Heizplatten. Die Positionierung von Rand und Mitte ist für den Fachmann bei einer Draufsicht auf die Anordnung der Heizplatten ersichtlich. Die mittleren Heizplatten sind bevorzugt jene Heizplatte, welche von äußeren Heizplatten eingefasst sind. Die sich am Rand befindenden Heizplatten haben keine Umrandung von weiteren äußeren Heizplatten. Eine leicht erhöhte Temperatur meint bevorzugt eine Temperaturdifferenz zwischen 0,1°C und 2°C besonders bevorzugt zwischen 0,1°C und 0,5°C.

In einer weiteren bevorzugten Ausführungsform der Erfindung ist die Vorrichtung dadurch gekennzeichnet, dass die Behandlungsfläche eine Dicke zwischen 0,2 mm und 5 mm, bevorzugt zwischen 0,5 mm und 2 mm, besonders bevorzugt zwischen 1 mm und 1,5 mm aufweist und aus einem Material besteht, welches eine Wärmeleitfähigkeit bei 50°C zwischen 20 W/mK und 400 W/mK, bevorzugt zwischen 100 und 350 W/mK aufweist. Die Wärmeleitfähigkeit (auch als Wärmeleitzahl bezeichnet) charakterisiert bevorzugt die thermischen Eigenschaften des Materials, aus welchem die Behandlungsfläche gefertigt ist. Die Wärmeleitfähigkeit gibt an, wie hoch die Wärmemenge ist, welche durch die Behandlungsfläche geleitet wird, wenn an dieser ein Temperaturgradient anliegt. Neben der Wärmeleitfähigkeit hängt der Wärmetransport von der Dicke der Behandlungsfläche, der Größe der Behandlungsfläche und dem Temperaturunterschied zwischen der Innenseite der Behandlungsfläche (Kontakt mit den Heizplatten) und der äußeren Seite der Behandlungsfläche (Kontakt mit der Haut) ab. Die Wärmeleitfähigkeit wird bevorzugt als Verhältnis der transportierten Wärmeleistung Watt (W) pro Temperaturdifferenz in Millikelvin (mK) angegeben. Da die Wärmeleitfähigkeit sich weiterhin in Abhängigkeit der Temperatur leicht ändern kann, wird vorliegend die Referenztemperatur mit 50°C angegeben. Die Dicke der Behandlungsfläche bezeichnet weiterhin bevorzugt die Ausdehnung der Behandlungsfläche zwischen der äußersten Fläche, welche die Haut kontaktiert, und der innersten Fläche, an welcher die Heizplatten anliegen.

Bei einer Dicke der Behandlungsfläche von zwischen 0,2 mm und 5 mm, bevorzugt zwischen 0,5 mm und 2 mm und besonders bevorzugt zwischen 1 mm und 1,5 mm ergibt sich in Kombination mit der bevorzugten Wärmeleitfähigkeit zwischen 100 und 350 W/mK eine therapeutisch besonders wirkungsvolle Wärmeabgabe an die Haut. In experimentellen Versuchen haben sich die bevorzugt genannten Parameter als überraschend vorteilhaft erwiesen. So vermeidet eine derart konzipierte Behandlungsfläche eine zu schnelle Abgabe der Wärme an die betreffenden Hautpartien, wodurch ein unangenehm stechender Schmerz ausgelöst werden könnte. Dennoch erfolgt die Wärmeabgabe in einem Zeitraum, welcher ausreichend impulshaft erfolgt, um effektiv die Rezeptoren anzuregen und einen Juckreiz zu überlagern. Die genannten Parameter stellen daher eine optimierte Auswahl dar, welche für einen Fachmann nicht nahelag.

In einer bevorzugten Ausführungsform umfasst die Behandlungsfläche Keramik oder Gold. Es ist besonders bevorzugt, dass die Behandlungsfläche aus Gold oder Keramik besteht. Die Materialien Keramik und Gold fallen zum einen in den experimentell ermittelten, bevorzugten Bereich der Wärmeleitfähigkeit. Darüber hinaus zeichnen sich sowohl Keramik als auch Gold auf überraschende Weise zur Behandlung von dermatologischem Juckreiz aus. Insbesondere entfalten die Materialien bei chronischem Pruritus, Allergien oder nach Kontakt mit giftigen Nesseltieren bei den Patienten eine gesteigert empfundene Schmerzüberlagerung. Dies ist insofern überraschend, als dass der Effekt über den reinen Temperatureffekt durch thermisch vergleichbare Materialien hinausgehen kann. Weiterhin zeichnen sich die Materialien bei der betreffenden Behandlungstemperatur durch eine überraschend antimikrobielle Funktion aus. Dieser Eigenschaft kommt bei Pruritus eine besondere Bedeutung zu. Insbesondere bei chronischem Pruritus oder großflächig betroffen Hautpartien, neigen die Patienten dazu durch Kratzen Scheuern oder Rubbeln eine kurzfristige, subjektive Linderung anzustreben. Durch psychologische Verstärkung kann dies zu einem zwanghaften Verhalten auswachsen. Als Nebenwirkung von Pruritus treten daher mikroskopischen Wunden, Kratzspuren, Hautläsionen oder auch offene Wunden gehäuft auf. Die antimikrobielle Funktion von Gold und Keramik bei der Behandlungstemperatur zeigt daher neben der Linderung des Juckreizes langfristig positive Effekte für die Erholung der Haut. Darüber hinaus zeichnen sich Gold und Keramik durch eine überraschende hohe biologische Verträglichkeit aus, welche gepaart mit einer besonders geringen Ausprägung von Allergien gegenüber diesen Materialien die Anwendung in einem Gerät zur Behandlung von vorwiegend dermatologischen Erkrankungen besonders auszeichnet. Eine besonders bevorzugte Keramik ist Aluminiumnitrid. Diese zeichnet sich in besonders starkem Maße durch eine außergewöhnliche biologische Verträglichkeit, überraschend antimikrobielle Funktion bei der Behandlungstemperatur und ausgezeichnete thermische Eigenschaften aus. Zudem ist eine Behandlungsfläche aus Aluminiumnitrid besonders stark elektrisch isolierend, sodass eine erhöhte Sicherheit in der Anwendung gewährleistet werden kann.

In einer weiteren bevorzugten Ausführungsform der Erfindung ist die Vorrichtung dadurch gekennzeichnet, dass die Vorrichtung zwischen 4 und 18 bevorzugt zwischen 8 und 14 Heizplatten umfasst und die Größe der Heizplatten zwischen 0,05 cm² und 2 cm², bevorzugt zwischen 0,1 cm² und 0,5 cm², beträgt. Es ist besonders bevorzugt, dass die Heizplatten kreisförmig sind, bevorzugt mit einem Durchmesser zwischen 3 und 10 mm. Weiterhin ist besonders bevorzugt, dass die Heizplatten gitterförmig angeordnet vorliegen, wobei ein bevorzugter Abstand zwischen den Heizplatten zwischen 0,2 mm und 10 mm, bevorzugt zwischen 0,5 mm und 3 mm, beträgt. Die Anordnung der Heizplatten führt zu einer überraschend starken Linderung des Juckreizes, wodurch insbesondere auch eine Behandlung von chronischem Pruritus, flächigen Allergien oder Quallenstichen besonders wirkungsvoll möglich wird. Weiterhin kommt es bei der bevorzugten Dimensionierung der Heizplatten zu einem synergistischen, positiven Effekt, wenn diese eine Behandlungsfläche erhitzen, welche zusammenhängend ausgestaltet ist, eine Dicke zwischen 0,5 mm und 2 mm aufweist und aus einem Material besteht mit einer Wärmeleitfähigkeit bei 50°C zwischen 20 und 400 W/mK, bevorzugt zwischen 100 und 350 W/mK. Für diese ganz besonders bevorzugte Ausführungsform ergibt sich eine optimale Wärmeübertragung. Die Wärmeleitfähigkeit in Verbindung mit der Dicke bestimmt nicht nur den Wärmetransport zwischen den Heizplatten und der äußeren Oberfläche der Behandlungsfläche, sondern zudem die Temperaturverteilung innerhalb der Ebene der Behandlungsfläche. Die genannten Parameter zeichnen sich dabei durch eine besonders wirkungsvolle Temperaturverteilung aus, welche hinreichend homogen ist, um eine gleichmäßige Wirkung auf den Hautpartien zu erreichen, aber dennoch eine leicht heterogene, punktuelle Wärmeabgabe erreicht. Hierbei liegt an der Position der Behandlungsfläche direkt unterhalb der jeweiligen Heizplatten eine leicht erhöhte Temperatur, welche bevorzugt um 0,1°C bis 2°C höher ist als der Durchschnitt der Behandlungstemperatur. Diese Verteilung ist auf überraschende Weise besonders wirkungsvoll zur Verminderung von Juckreiz.

In einer bevorzugten Ausführungsform der Erfindung umfasst die Vorrichtung mindestens einen Temperatursensor zur Messung der Temperatur der Behandlungsfläche und/oder mindestens einer Heizplatte, wobei die Steuervorrichtung basierend auf Messdaten der Temperatursensoren die Temperatur der mindestens einen Heizplatte einstellt. Ein Temperatursensor bezeichnet bevorzugt ein elektrisches oder elektronisches Bauelement, welches durch Messung der Temperatur ein elektrisches Signal an die Steuervorrichtung sendet.

Im Stand der Technik sind eine Vielzahl von Temperatursensoren bekannt wie bspw. Halbleiter-Temperatursensoren, Widerstandstemperatursensoren, Pyroelektrische Materialien, Thermoelemente oder Schwingquarze. Die Steuervorrichtung ist weiterhin bevorzugt derart konfiguriert, dass diese die Messwerte der Temperatursensoren aufnehmen und auswerten kann, um eine Regulation der Heizplatten zu bewirken. Die Regulation der Heizplatten kann bevorzugt mithilfe des Anliegens eines elektrischen Stroms oder einer Spannung erfolgen. Es ist besonders bevorzugt, dass der Temperatursensor die Temperatur der Behandlungsfläche direkt misst, d.h. dass der Temperatursensor in Kontakt mit der Behandlungsfläche ist, wobei sich der Temperatursensor sowohl auf der inneren Seite der Behandlungsfläche, als auch auf der äußeren Seite der Behandlungsfläche befinden kann. Im Falle einer zusammenhängenden Behandlungsfläche kann es bevorzugt sein, dass mehrere Temperatursensoren an unterschiedlichen Positionen der Behandlungsfläche angebracht vorliegen, um ein besonders präzise Auslesen der ortsabhängigen Temperaturverteilung zu ermöglich. Es kann aber auch bevorzugt sein, dass der Temperatursensor nicht die Behandlungsfläche, sondern die Heizplatten kontaktiert und überwacht. Aufgrund der Kenntnis der materiellen Eigenschaften der Behandlungsfläche kann vorteilhafterweise aus den Messdaten über die Heizplatten ebenfalls auf die Temperaturverteilung der Behandlungsfläche geschlossen werden. Eine Auswertung der Temperatur der Heizplatten sowie der Temperatur der Behandlungsfläche erlaubt eine besonders präzise Regulation der Heizplatten, um die optimale Temperaturverteilung und Wärmeübertragung sicher zu stellen. Zu diesem Zweck kann es sowohl bevorzugt sein einzelne Heizplatten separat anzusteuern, beispielsweise um ein Temperaturgefälle auszugleichen. Es kann aber auch bevorzugt sein, dass die Heizplatten gemeinsam gesteuert werden, sodass eine Temperaturregulation für alle Heizplatten gleichmäßig erfolgt. Insbesondere im Hinblick auf die vielfältigen Anwendungsmöglichkeiten der Vorrichtung zur Behandlung verschiedener Pruritus-Erkrankungen, ist eine temperaturbasierte Feedback-Regulation geeignet, um den unterschiedlichen Sicherheitsanforderungen zu genügen. Im Falle der Behandlung von Hautpartien mit Läsionen oder bei Kindern ist es von besonders hoher Wichtigkeit eine exakte Behandlungstemperatur einzustellen und einen Maximalwert nicht zu überschreiten. Eine Feedbackkontrolle basierend auf Temperaturmessungen kann darüber hinaus Bedienungsfehler wirksam vorbeugen. Durch die Verwendung von mindestens einem Temperatursensor wird somit nicht nur die Wirksamkeit, sondern zudem die Sicherheit und Zuverlässigkeit der Vorrichtung erhöht.

In einer bevorzugten Ausführungsform der Erfindung weist die Vorrichtung eine Software und/oder Hardware basierte Sicherheitsabschaltung bei Überschreiten einer Maximaltemperatur auf. Diese bevorzugte Ausführungsform erlaubt einen besonderen sicheren Betrieb der Vorrichtung. Aufgrund der im Vergleich zum Stand der Technik ungleich größeren Behandlungsfläche ist eine Sicherheitsabschaltung in besonderem Maße vorteilhaft. Gerade bei der Behandlung großflächigen Hautpartien kann es andernfalls durch Systemfehler zu Hautschädigungen kommen. Weiterhin ist die breite Anwendbarkeit der Vorrichtung zu beachten, welche neben unterschiedlich sensitiven Hautpartien auch verschiedensten Patientenkollektive von Kindern, über Senioren bis hin zu Schwangeren umfasst. Eine Software und/oder Hardware implementierte Abriegelung der Vorrichtung durch eine Sicherheitsschaltung ab einer Maximaltemperatur kann höchsten Sicherheitsanforderungen Genüge leisten. Es ist besonders bevorzugt, dass die Sicherheitsabschaltung Hardware implementiert ist, bspw. durch Schmelzsicherungen oder Bimetallstreifen, sodass ohne ein aktives Eingreifen der Steuervorrichtung das Überschreiten einer Maximaltemperatur vermieden wird. Es kann darüber hinaus bevorzugt sein, dass zudem eine softwarebasierte Abschaltung erfolgt. Diese wird bevorzugt durch die Steuervorrichtung umgesetzt, welche die Temperaturdaten der Heizplatten und/oder der Behandlungsfläche auswertet und mit einem Maximalwert vergleicht.

In einer weiteren bevorzugten Ausführungsform der Erfindung umfasst die Vorrichtung eine Schmelzsicherung, welche bei Kurzschluss oder ungeregeltem Durchheizen die Vorrichtung abschaltet. Eine Schmelzsicherung ist bevorzugt eine Vorrichtung zum Schutze eines Überstromes, wobei durch das Abschmelzen eines Schmelzleiters der Stromkreis unterbrochen wird. Somit kann eine durch den Schmelzleiter vorgegebene Stromstärke nicht überschritten werden. Eine Schmelzsicherung verhindert effektiv einen Kurzschluss und kann zudem ein ungeregeltes Durchheizen der Vorrichtung verhindern. Ein ungeregeltes Durchheizen der Vorrichtung bezeichnet bevorzugt ein aufgrund von Software- oder Hardwarefehlern erfolgtes Heizen der Heizplatten über die Maximaltemperatur hinaus. Ein solches ungeregeltes Durchheizen kann beispielsweise auftreten, falls die Temperatursensoren defekt sind oder aber eine Sicherheitsabschaltung nicht ordnungsgemäß einspringt. Durch die Schmelzsicherung kann eine zusätzliche hardwarebasierte Sicherung erreicht werden. Mithilfe einer Schmelzsicherung kann somit ein weiteres technisches Merkmal bereitgestellt werden, welches die Sicherheit der Vorrichtung insbesondere im Hinblick auf die Anforderungen durch den vielfältigen medizinischen Einsatz erhöht.

In einer weiteren Ausführungsform der Erfindung ist die Vorrichtung dadurch gekennzeichnet, dass die Behandlungstemperatur während der Behandlungsphase in einem Bereich von ±10% bevorzugt ± 5% und ganz bevorzugt ± 3% gehalten werden kann. Durch das bevorzugt konstante Halten der Behandlungstemperatur in den vorgenannten Bereichen kann eine besonders präzise und konstante Wärmeübertragung sichergestellt werden, sodass eine optimierte Wärmeübertragung auf die betreffende Hautstellen gewährleistet wird. Zur Einhaltung der genannten Parameter können bevorzugt Messdaten der Temperatursensoren genutzt und ausgewertet werden.

In einer weiteren bevorzugten Ausführungsform der Erfindung ist die Vorrichtung dadurch gekennzeichnet, dass die Vorrichtung eine optische Anzeige und/oder einen Schallgeber umfasst, welcher den Start der Aufheizphase, das Erreichen der Behandlungstemperatur, die Dauer der Behandlungsphase und/oder das Ende der Behandlungsphase durch ein akustisches und/oder optisches Signal anzeigt. Die optische Anzeige kann bevorzugt durch Leuchtdioden (LED), Glühlampen, Flüssigkeitskristall (LCD) Displays oder andere bekannte optischen Anzeigen erfolgen. Bevorzugt kommt ein Farbcode zum Tragen, welcher an die Funktion angepasst ist. Beispielsweise kann die Aufheizphase durch ein oranges Signal angezeigt werden, die Behandlungsphase durch ein rotes Signal und das Ende der Behandlungsphase durch ein grünes Signal. Die akustische Signalgebung erfolgt bevorzugt durch einen Lautsprecher, welcher besonders bevorzugt kurze oder längere Pieptöne aussendet. Durch die optische und/oder akustische Signalgebung erfährt der Nutzer zu jeder Zeit der Vorbereitungs- oder Behandlungsphase den Zustand der Vorrichtung. Überraschenderweise ergibt sich dadurch eine zusätzlich psychologische Wirkung, welche durch eine Konzentration auf die Signalgebung zu einer noch stärkeren Minderung des Juckreizes führt. Weiterhin wird durch die bevorzugte Ausführungsform die Bedienungsfreundlichkeit und -sicherheit sowie die Compliance der Patienten in großem Maße gesteigert. Zudem erlaubt die optische und/oder akustische Signalgebung weitere Sicherheitsmechanismen einzuführen. So kann das Überschreiten einer Maximaltemperatur dem Nutzer schnell und deutlich angezeigt werden. Hierdurch kann der Benutzer die Behandlungsfläche von der Hautpartie entfernen, bevor Hautschädigungen auftreten können. Es hat sich gezeigt, dass die Reaktion durch Schmerzempfindungen zum Eigenschutz deutlich langsamer ist, als die Reaktion auf ein optisches und/oder akustisches Warnsignal. Auch führen Warnsignale zu einem effektiveren und schnelleren Schutz, als dies durch eine Sicherheitsabschaltung der Heizplatten möglich ist.

In einer weiteren bevorzugten Ausführungsform betrifft die Erfindung eine Verwendung der Vorrichtung zur Behandlung von chronischem Pruritus, zur Verminderung von Juckreiz bei Dermatitis oder nach Kontakt mit giftigen Nesseltieren oder Pflanzen, bevorzugt nach Kontakt mit Quallen. Darüberhinaus kann die Vorrichtung zur Behandlung von Pruritus als Nebenwirkung von Hauterkrankungen wie Ekzeme, Neurodermitis, Urtikaria, Hautinfektionen, Xerodermie, von Parasiten (z.B. Skabies), von Stoffwechselerkrankungen oder Infektionskrankheiten eingesetzt werden. Auch bei Allergien gegenüber Lebensmitteln oder Medikamenten kann die Vorrichtung wirksam den Juckreiz vermindern. Erfindungsgemäß wurde erkannt, dass eine hyperthermische Behandlung mit den bevorzugten genannten Parametern für die Behandlungstemperatur sowie Dimensionierung der Behandlungsfläche in den genannten Anwendungsgebieten zu einem überraschend positiven Effekt führt. Beispielsweise kann die Vorrichtung im Vergleich zu konventionellen Salben oder Cremen deutlich schneller und wirksamer Juckreiz nach Kontakt mit giftigen Nesseltieren vorbeugen. Bei der Verwendung der Vorrichtung wird diese bevorzugt auf die Hautpartien aufgelegt. Anschließend erfolgt bevorzugt nach einer Eingabe durch den Nutzer automatisiert die Regulation der Temperatur der Behandlungsfläche mit Hilfe der Steuervorrichtung. Vorteilhafterweise muss hierfür kein Arzt zugegen sein, sondern die Person kann selbstständig die Vorrichtung nutzen. Aufgrund der einfachen Handhabung und der nahezu sofort spürbaren Linderung des Juckreizes ist die Compliance ungewöhnlich hoch. Durch die ausgeprägte Therapietreue lassen sich auch schwerwiegende Pruritus-Erkrankungen nachhaltig behandeln. Weiterhin erlaubt die kompakte Größe einen flexiblen Einsatz. Die Vorrichtung kann problemlos transportiert und somit auch vorbeugend bei Reisen mitgeführt werden, bei denen ein erhöhtes Risiko von Allergien oder Quallenstichen besteht. Der dadurch ermöglichte unmittelbare Beginn einer Behandlung nach Auftreten der ersten Symptome des Pruritus wirkt sich positiv auf den Behandlungserfolg aus.

Im Folgenden soll die Erfindung an Hand von Beispielen näher erläutert werden, ohne auf diese beschränkt zu sein.

### Kurzbeschreibung der Abbildungen

- Fig. 1: Schematische Seitenansicht einer bevorzugten Ausführungsform der Vorrichtung
- Fig. 2: Schematische Draufsicht auf eine bevorzugte Behandlungsfläche und Anordnung der Heizplatten

### Detaillierte Beschreibung der Abbildungen

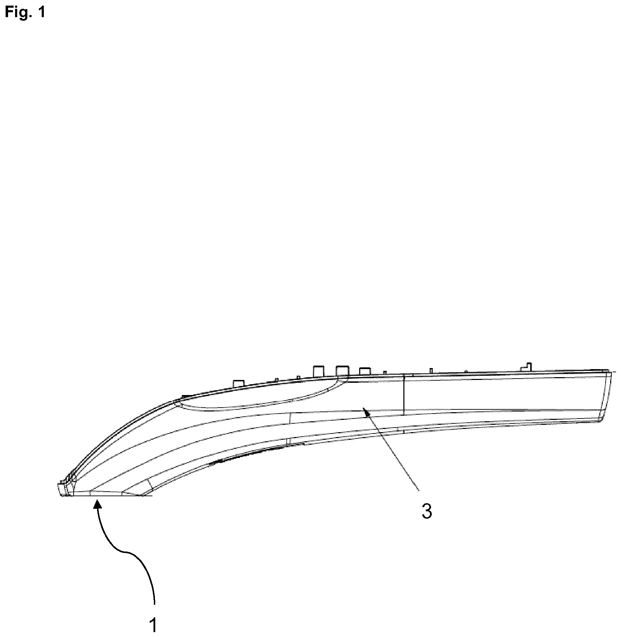

Figur 1 zeigt eine schematische Seitenansicht einer bevorzugten Ausführungsform der Vorrichtung. Das Gehäuse (3) der Vorrichtung ist bevorzugt derart ausgestaltet, dass dieses griffig in der Hand liegt, um eine einfache und bequeme Handhabung zu gewährleisten. Die bevorzugte Ausführungsform des Gehäuses (3) umfassend einen Griff ist auf besondere Weise geeignet, um verschiedene Körperstellen ohne Mühen mit der Behandlungsfläche (1) zu erreichen. Die Behandlungsfläche (1) ist hierzu auf der Unterseite des Gehäuses in einem vorderen Bereich eingebettet.

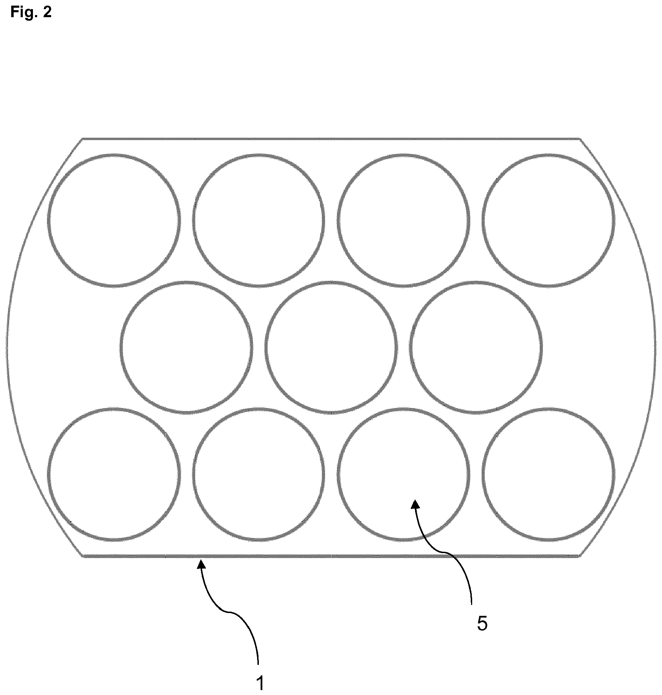

Figur 2 zeigt eine schematische Ansicht einer bevorzugten Behandlungsfläche (1) und Anordnung der Heizplatten (5). Zur besseren Kenntlichkeit ist die Behandlungsfläche (1) nur durch deren Umrandung dargestellt, sodass die Anordnung der sich in der Vorrichtung befindlichen Heizplatten (5) sichtbar wird. In der besonders bevorzugten Ausführungsform umfasst die Vorrichtung 11 Heizplatten (5) mit einem Durchmesser von ca. 6 mm. Die Behandlungsfläche (1) wird durch die kontaktierenden Heizplatten (5) auf die Behandlungstemperatur erhitzt und besteht in dieser besonders bevorzugten Ausführungsform aus der Keramik Aluminiumnitrid mit einer Dicke von 1,3 mm. Mit dieser besonders bevorzugten Ausführungsform der Vorrichtung kann besonders effektiv der Juckreiz bei einer Vielzahl von Erkrankungen vermindert werden.

Es wird darauf hingewiesen, dass verschiedene Alternativen zu den beschriebenen Ausführungsformen der Erfindung verwendet werden können, um die Erfindung auszuführen und zu der erfindungsgemäßen Lösung zu gelangen. Die erfindungsgemäße Vorrichtung sowie deren Verwendung beschränken sich in ihren Ausführungen somit nicht auf die vorstehenden bevorzugten Ausführungsformen. Vielmehr ist eine Vielzahl von Ausgestaltungsvarianten denkbar, welche von der dargestellten Lösung abweichen können. Ziel der Ansprüche ist es, den Schutzumfang der Erfindung zu definieren. Der Schutzumfang der Ansprüche ist darauf gerichtet, die erfindungsgemäße Vorrichtung und bevorzugte Verwendungen sowie äquivalente Ausführungsformen von diesen abzudecken.

### BEZUGSZEICHENLISTE

- 1: Behandlungsfläche
- 3: Gehäuse
- 5: Heizplatte

### REFERENZEN

Cohen MR, Moiseenkova-Bell VY. Structure of thermally activated TRP channels. Curr Top Membr. 2014;74:181-211.
Mergler S et al. Temperature-sensitive transient receptor potential channels in corneal tissue layers and cells. Ophthalmic Res. 2014;52(3):151-9.
Matterne U, Apfelbacher CJ, Loerbroks A, Schwarzer T, Büttner M, Ofenloch R, Diepgen TL, Weisshaar E: Prevalence, Correlates and Characteristics of Chronic Pruritus: A Population-Based Cross-sectional Study. Acta Derm Venereol 2011; 91: 674-679
Rothmann S. Physiology of itching. Physiol Rev 1941; 21: 357-381
Ständer S, Weisshaar E, Mettang T, Szepietowski JC, Carstens E, Ikoma A, Bergasa N, Gieler U, Misery L, Wallengren J, Darsow U, Streit M, Metze D, Luger TA, Greaves MW, Schmelz M, Yosipovitch G, Bernhard J. Clinical classification of itch: a position paper of the International Forum for the Study of Itch. Acta Dermatol Venerol 2007, 87: 291-294
Ständer S, Schäfer I, Phan NQ, Blome C, Herberger K, Heigel H, Augustin M.: Prevalence of chronic pruritus in Germany: Results of a cross-sectional study in a sample working population of 11,730. Dermatology 2010;221:229-35
Somogyi CS et al. Polymodal Transient Receptor Potential Vanilloid (TRPV) Ion Channels in Chondrogenic Cells. Int J Mol Sci. 2015;16(8): 18412-38.
Vogelgsang L, Loerbroeks A, Apfelbacher CJ, et al. Incidence of chronic pruritus and its determinants: results from a population-based study. Exp Dermatol 2012;21:e1-e48
Yao J, Liu B, Qin F. Modular thermal sensors in temperature-gated transient receptor potential (TRP) channels. Proc Natl Acad Sci USA. 2011;108(27):11109-14.

## Patentansprüche

1. Vorrichtung zur hyperthermischen Behandlung von Juckreiz umfassend
a) eine Behandlungsfläche (1)
b) mindestens eine Heizplatte (5)
und
c) eine Steuervorrichtung zur Regulation der Temperatur der Behandlungsfläche (1) wobei die Steuervorrichtung konfiguriert ist die Temperatur der mindestens einen Heizplatte (5) zu regulieren, sodass die Behandlungsfläche (1) in einer Aufheizphase auf eine Behandlungstemperatur zwischen 42°C und 53°C reguliert wird und
**dadurch gekennzeichnet, dass**
die Behandlungstemperatur in einer Behandlungsphase für einen zusammenhängenden Zeitraum von 4 sec bis 6 sec gehalten wird, und wobei die Größe der Behandlungsfläche (1) zwischen 6 cm² und 18 cm² beträgt.

2. Vorrichtung gemäß dem vorherigen Anspruch
**dadurch gekennzeichnet, dass**
durch die Regulation der Behandlungsfläche auf die Behandlungstemperatur in der Behandlungsphase ein Hitzeimpuls generiert wird, welcher es erlaubt eine wohldefinierte Wärmemenge auf eine Hautstelle zu bringen.

3. Vorrichtung gemäß einem der vorherigen Ansprüche
**dadurch gekennzeichnet, dass**
Steuervorrichtung ein Prozessor oder ein Prozessorchip ist.

4. Vorrichtung gemäß einem der vorherigen Ansprüche
**dadurch gekennzeichnet, dass**
die Behandlungstemperatur zwischen 50°C und 53°C beträgt.

5. Vorrichtung gemäß einem der vorherigen Ansprüche
**dadurch gekennzeichnet, dass**
die Vorrichtung mindestens zwei Heizplatten (5), bevorzugt mindestens vier Heizplatten (5) und ganz besonders bevorzugt mindestens sechs Heizplatten (5) umfasst, welche in Kontakt mit der Behandlungsfläche (1) vorliegen und deren Temperatur durch die Steuervorrichtung eingestellt werden kann, wobei durch ein Erhitzen der Heizplatten (5) eine Einstellung der Behandlungstemperatur an der Behandlungsfläche (1) erfolgt.

6. Vorrichtung gemäß einem der vorherigen Ansprüche
**dadurch gekennzeichnet, dass**
die Behandlungsfläche (1) eine zusammenhängende Fläche ist und/oder die Behandlungsfläche (1) aus mehreren Teilflächen besteht, welche bevorzugt kongruent zu den Heizplatten (5) sind.

7. Vorrichtung gemäß dem vorherigen Anspruch
**dadurch gekennzeichnet, dass**
die Steuervorrichtung die Heizplatten (5) in der Aufheizphase auf eine Heiztemperatur zwischen 43°C und 54°C regulieren kann und die Heiztemperatur in der Behandlungsphase für einen Zeitraum zwischen 4 sec und 6 sec gehalten werden kann.

8. Vorrichtung gemäß einem der vorherigen Ansprüche
**dadurch gekennzeichnet, dass**
die Behandlungsfläche (1) eine Dicke zwischen 0,2 mm und 5 mm, bevorzugt zwischen 0,5 mm und 2 mm und besonders bevorzugt zwischen 1 mm und 1,5 mm aufweist und aus einem Material besteht, welches eine Wärmeleitfähigkeit bei 50°C zwischen 20 und 400 W/mK, bevorzugt zwischen 100 und 350 W/mK aufweist.

9. Vorrichtung gemäß einem der vorherigen Ansprüche
**dadurch gekennzeichnet, dass**
die Behandlungsfläche (1) Keramik und/oder Gold umfasst.

10. Vorrichtung gemäß einem der vorherigen Ansprüche
**dadurch gekennzeichnet, dass**
die Vorrichtung zwischen 4 und 18 bevorzugt zwischen 8 und 14 Heizplatten (5) umfasst und die Größe der Heizplatten (5) zwischen 0,05 cm² und 2 cm², bevorzugt zwischen 0,1 cm² und 0,5 cm², beträgt.

11. Vorrichtung gemäß einem der vorherigen Ansprüche
**dadurch gekennzeichnet, dass**
die Vorrichtung mindestens einen Temperatursensor zur Messung der Temperatur der Behandlungsfläche (1) und/oder mindestens einer Heizplatte (5) umfasst und die Steuervorrichtung basierend auf Messdaten des Temperatursensors die Temperatur der mindestens einen Heizplatte (5) einstellt.

12. Vorrichtung gemäß einem der vorherigen Ansprüche
**dadurch gekennzeichnet, dass**
die Vorrichtung einen Software und/oder Hardware basierte Sicherheitsabschaltung bei Überschreiten einer Maximaltemperatur aufweist.

13. Vorrichtung gemäß einem der vorherigen Ansprüche
**dadurch gekennzeichnet, dass**
die Vorrichtung eine Schmelzsicherung umfasst, welche bei Kurzschluss oder ungeregeltem Durchheizen die Vorrichtung abschaltet.

14. Vorrichtung gemäß einem der vorherigen Ansprüche
**dadurch gekennzeichnet, dass**
die Behandlungstemperatur während der Behandlungsphase in einem Bereich von ±10% bevorzugt ± 5% und ganz bevorzugt ± 3% gehalten werden kann.

15. Vorrichtung gemäß einem der vorherigen Ansprüche
**dadurch gekennzeichnet, dass**
die Vorrichtung eine optische Anzeige und/oder einen Schallgeber umfasst, welcher den Start der Aufheizphase, das Erreichen der Behandlungstemperatur, die Dauer der Behandlungsphase und/oder das Ende der Behandlungsphase durch ein akustisches und/oder optisches Signal anzeigt und/oder
welcher das Nichterreichen einer vorgegebenen Behandlungstemperatur und/oder Abweichungen der Behandlungstemperatur während der Behandlungsphase, welche größer als bevorzugt ± 5% und ganz bevorzugt ± 3% sind, durch eine optisches und/oder akustisches Warnsignal anzeigt.

## Claims

1. Device for hyperthermal treatment of itching, comprising
a) a treatment surface (1)
b) at least one heating plate (5)
and
b) a control device to regulate the temperature of the treatment surface (1) wherein the control device is configured to regulate the temperature of the at least one heating plate (5) such that in a heating phase, the treatment surface (1) is regulated to a treatment temperature between 42° C und 53° C, and
**characterized in that**
in a treatment phase, the treatment temperature is maintained continuously for a duration of 4 seconds to 6 seconds and wherein the size of the treatment surface (1) is between 6 cm² and 18 cm².

2. Device according to the preceding claim
**characterized in that**
by regulating the treatment surface to the treatment temperature for the treatment phase, a heating pulse is generated, which enables a well-defined quantity of heat to be brought to an area of the skin.

3. Device according to one of the preceding claims
**characterized in that**
the control device is a processor or processor chip.

4. Device according to any one of the preceding claims
**characterized in that**
the treatment temperature is between 50° C and 53° C.

5. Device according to one of the preceding claims
**characterized in that**
the device is **characterized in that** the device is comprised of at least two heating plates (5), preferably at least four heating plates (5) and very especially preferred at least six heating plates (5), which are present with the treatment surface and whose temperature can be set by the control device, whereby the setting of the treatment temperature on the treatment surface (1) occurs through heating of the heating plates (5).

6. Device according to one of the preceding claims
**characterized in that**
the treatment surface (1) is a connected surface and/or the treatment surface (1) consists of several partial surfaces, which preferentially are congruent to the heating plates (5).

7. Device according to one of the preceding claims
**characterized in that**
the control device can regulate the heating plates (5) in the heating phase to a heating temperature between 43° C and 54° C and can maintain the heating temperature in the treatment phase for a time period between 4 seconds and 6 seconds.

8. Device according to one of the preceding claims
**characterized in that**
the treatment surface (1) has a thickness of between 0.2 mm and 5 mm, preferentially between 0.5 mm and 2 mm, and especially preferentially between 1 mm and 1.5 mm, and is made of a material that can conduct heat at 50°C between 20 and 400 W/mK, preferentially between 100 and 350 W/mk.

9. Device according to one of the preceding claims
**characterized in that**
the treatment surface (1) is made of ceramics and/or gold.

10. Device according to one of the prior claims
**characterized in that**
the device comprises between 4 and 18, preferably between 8 and 14, heating plates (5), and the size of the heating plates (5) lies between 0.05 cm² and 2 cm², preferentially between 0.1 cm² und 0.5 cm².

11. Device according one of the preceding claims
**characterized in that**
the device comprises at least one temperature sensor to measure the temperature of the treatment surface (1) and/or at least one heating plate (5), and the control device sets the temperature of at least one heating plate (5) based on measurement data of the temperature sensor.

12. Device according one of the preceding claims
**characterized in that**
the device has a safety shut off based on a software program and/or hardware that occurs when a maximum temperature is exceeded.

13. Device according one of the preceding claims
**characterized in that**
the device includes a fuse that turns off the device if there is a short circuit or unregulated heating.

14. Device according to one of the prior claims
**so characterized in that**
the treatment temperature during the treatment phase can be maintained in a range of +/-10%, preferred +/- 5%, and especially preferred +/-3%.

15. Device according to one of the prior claims
**so characterized in that**
the device has an optical display and/or a sound emitter, which by an acoustical and/or optical signal indicate the start of the heating up phase, reaching the treatment temperature, the duration of the treatment phase, and/or the end of the treatment phase
and/or which indicate by an optical and/or acoustical warning signal of not reaching a preset treatment temperature and/or deviations from the treatment temperature during the treatment phase that is greater than the preferred +/-5% and especially preferred +/-3%.

## Revendications

1. Dispositif pour le traitement hyperthermique des démangeaisons comprenant
a) une surface de traitement (1)
b) au moins une plaque chauffante (5)
et
c) un dispositif de commande pour réguler la température de la surface de traitement (1), dans lequel
le dispositif de commande est configuré pour réguler la température de l'au moins une plaque chauffante (5), de sorte que la surface de traitement (1) est régulée à une température de traitement comprise entre 42 °C et 53 °C dans une phase de chauffage et
**caractérisé en ce que** la température de traitement est maintenue dans une phase de traitement pendant une période continue de 4 secondes à 6 secondes, et dans lequel
la taille de la surface de traitement (1) est comprise entre 6 cm² et 18 cm².

2. Dispositif selon la revendication précédente,
**caractérisé en ce que**
par la régulation de la surface de traitement à la température de traitement, une impulsion de chaleur est générée pendant la phase de traitement, permettant d'apporter une quantité bien définie de chaleur à une zone de la peau.

3. Dispositif selon l'une des revendications précédentes,
**caractérisé en ce que**
le dispositif de commande est un processeur ou une puce de processeur.

4. Dispositif selon l'une des revendications précédentes,
**caractérisé en ce que**
la température de traitement est comprise entre 50 °C et 53 °C.

5. Dispositif selon l'une des revendications précédentes,
**caractérisé en ce que**
le dispositif comprend au moins deux plaques chauffantes (5), de préférence au moins quatre plaques chauffantes (5) et de manière tout particulièrement préférée au moins six plaques chauffantes (5), qui sont en contact avec la surface de traitement (1) et dont la température peut être réglée par le dispositif de commande, dans lequel la température de traitement sur la surface de traitement (1) est ajustée par le chauffage des plaques chauffantes (5).

6. Dispositif selon l'une des revendications précédentes,
**caractérisé en ce que**
la surface de traitement (1) est une surface continue et/ou la surface de traitement (1) est constituée de plusieurs surfaces partielles qui sont de préférence congruentes aux plaques chauffantes (5).

7. Dispositif selon l'une des revendications précédentes,
**caractérisé en ce que**
le dispositif de commande peut réguler les plaques chauffantes (5) à une température de chauffage comprise entre 43°C et 54°C pendant la phase de chauffage, et la température de chauffage peut être maintenue pendant une durée comprise entre 4 secondes et 6 secondes pendant la phase de traitement.

8. Dispositif selon l'une des revendications précédentes,
**caractérisé en ce que**
la surface de traitement (1) a une épaisseur comprise entre 0,2 mm et 5 mm, de préférence entre 0,5 mm et 2 mm et de manière particulièrement préférée entre 1 mm et 1,5 mm et est constituée d'un matériau qui présente une conductivité thermique à 50 °C comprise entre 20 et 400 W/mK, de préférence entre 100 et 350 W/mK.

9. Dispositif selon l'une des revendications précédentes,
**caractérisé en ce que**
la surface de traitement (1) est en céramique et/ou en or.

10. Dispositif selon l'une des revendications précédentes,
**caractérisé en ce que**
le dispositif comprend entre 4 et 18, de préférence entre 8 et 14 plaques chauffantes (5) et la taille des plaques chauffantes (5) est comprise entre 0,05 cm² et 2 cm², de préférence entre 0,1 cm² et 0,5 cm².

11. Dispositif selon l'une des revendications précédentes,
**caractérisé en ce que**
le dispositif comprend au moins un capteur de température pour mesurer la température de la surface de traitement (1) et/ou au moins une plaque chauffante (5) et le dispositif de commande règle la température de l'au moins une plaque chauffante (5) sur la base de données de mesure provenant du capteur de température.

12. Dispositif selon l'une des revendications précédentes,
**caractérisé en ce que**
l'appareil dispose d'un arrêt de sécurité logiciel et/ou matériel lorsqu'une température maximale est dépassée.

13. Dispositif selon l'une des revendications précédentes,
**caractérisé en ce que**
le dispositif comprend un fusible qui éteint l'appareil en cas de court-circuit ou d'échauffement non régulé.

14. Dispositif selon l'une des revendications précédentes,
**caractérisé en ce que**
la température de traitement peut être maintenue pendant la phase de traitement dans une plage de ± 10 %, de préférence ± 5 % et de manière tout à fait préférée ± 3 %.

15. Dispositif selon l'une des revendications précédentes,
**caractérisé en ce que**
le dispositif comprend un affichage visuel et/ou une sirène, qui indique le début de la phase de chauffage, l'atteinte de la température de traitement, la durée de la phase de traitement et/ou la fin de la phase de traitement par un signal acoustique et/ou visuel et/ou
qui indique la non-atteinte d'une température de traitement prédéterminée et/ou des écarts de température de traitement au cours de la phase de traitement qui sont supérieurs de préférence à ± 5 % et de manière tout à fait préférée à ± 3 %, au moyen d'un signal d'avertissement visuel et/ou acoustique.
